# EUROPÄISCHE PATENTSCHRIFT

(11) **EP 2 349 218 B1**
(45) Veröffentlichungstag und Bekanntmachung des Hinweises auf die Patenterteilung: **18.09.2013**
(21) Anmeldenummer: 09783669.6
(22) Anmeldetag: 02.10.2009
(51) Int. Cl.: A61K 9/16, A61K 9/20

(54) **VERFAHREN ZUR HERSTELLUNG ORALER DARREICHUNGSFORMEN MIT KONTROLLIERTER FREISETZUNG**
METHOD FOR PRODUCING CONTROLLED-RELEASE ORAL DOSAGE FORMS
PROCÉDÉ DE FABRICATION DE FORMES POSOLOGIQUES ORALES À LIBÉRATION CONTRÔLÉE

(30) Priorität: 07.10.2008 EP 08166013
(43) Veröffentlichungstag der Anmeldung: 03.08.2011
(73) Patentinhaber: BASF SE, 67056 Ludwigshafen (DE)
(72) Erfinder: KOLTER, Karl, 67117 Limburgerhof (DE); MASCHKE, Angelika, 93047 Regensburg (DE)
(86) Internationale Anmeldenummer: PCT/EP2009/062800
(87) Internationale Veröffentlichungsnummer: WO 2010/040686

(56) Entgegenhaltungen:
- EP-A2- 1 166 776
- DE-A1- 3 612 212
- ANONYMOUS: "Placement of additives into a fluid stream" RESEARCH DISCLOSURE, MASON PUBLICATIONS, HAMPSHIRE, GB, Bd. 468, Nr. 113, 1. April 2003 (2003-04-01), XP007132555 ISSN: 0374-4353
- "Kollicoat" In: E.M. Hoepfner, A. Reng, P.C. Schmidt: "Fiedler Lexikon der Hilfstoffe", 31 December 2012 (2012-12-31), Editio Cantor Verlag Aulendorf vol. 5, pages 977-978,

## Beschreibung

Die vorliegende Erfindung betrifft ein Verfahren gemäß Anspruch 1 zur Herstellung fester oraler Darreichungsformen mit kontrollierter Wirkstofffreisetzung, enthaltend mindestens einen Wirkstoff, eine vorformulierte Mischung aus Polyvinylacetat und Polyvinylpyrrolidon, gegebenenfalls wasserlösliche Polymere oder lipophile Zusatzstoffe sowie gegebenenfalls weitere übliche Hilfsstoffe, dadurch gekennzeichnet, dass die Verarbeitung dieser Mischung oder Teilen dieser Mischung in einem Extruder bei Temperaturen zwischen 80 und 180°C erfolgt, wobei die Extrusion bei einem Druck von 2-25 MPa erfolgt.

Sogenannte Depot- oder Retardarzneiformen mit kontrollierter Freisetzung ("Controlled Release")gewinnen in der Arzneimitteltherapie immer größere Bedeutung infolge einer verbesserten, länger anhaltenden therapeutischen Wirkung und der Tatsache , dass die Darreichungsform weniger häufig verabreicht werden muss. Neben den Coatingretardformen, bei denen die Freisetzung durch einen Überzug gesteuert wird, finden immer häufiger Matrixformen Anwendung, bei denen der Wirkstoff in eine Grundlage eingebettet vorliegt, aus der er bei Kontakt mit Magen- oder Darmsaft langsam herausdiffundiert. Diese Formen lassen sich durch verschiedene Technologien herstellen wie z.B. Direkttablettierung oder Feuchtgranulierung. Häufig werden als Matrixbildner erodierbare Stoffe oder Gelbildner wie Hydroxypropylmethylcellulose oder Xanthan eingesetzt, die bei Kontakt mit wässrigen Medien die Freisetzung steuern.

Nachteile dieser Produkte und Verfahren ist, dass die Freisetzung stark abhängig ist von der Art der Granulierung, der Teilchengröße der Einsatzstoffe und Granulatkörnchen, dem Pressdruck und vom Salzgehalt bzw. der Osmolarität des Freisetzungsmediums. Häufig treten Freisetzungsschwankungen von Tablette zu Tablette und Charge zu Charge auf. Ferner weisen solche Tabletten häufig eine unzureichende mechanische Stabilität auf, das heißt eine niedrige Bruchfestigkeit und eine hohe Friabilität.

In der EP-A 1166776 ist ein Verfahren zur Herstellung von oralen Darreichungsformen mit retardierender Wirkung beschrieben, bei dem eine vorformulierte Mischung aus Polyvinylacetat und Polyvinylpyrrolidon mit Wirkstoffen durch Erwärmung auf 40 - 130°C granuliert wird. Die Granulation wird dadurch bewirkt, dass das Polyvinylacetat zu einer klebrigen Masse erweicht, wodurch die Pulverteilchen zu Granulatteilchen zusammenkleben. Hiermit wurden recht gute Ergebnisse hinsichtlich der Freisetzung des Wirkstoffes und der mechanischen Stabilität der Darreichungsform erzielt. Dennoch weisen die so erhaltenen Granulate gewisse Nachteile auf, beispielsweise hinsichtlich der Granulatgröße und der Porosität der gebildeten Granulatkörnchen, welche die Freisetzung beeinflussen. Dadurch wird der Prozess schwerer steuerbar.

In der EP-A 1138321 werden ebenfalls Tabletten aus einer vorformulierten Mischung aus Polyvinylacetat und Polyvinylpyrrolidon beschrieben, die durch Direkttablettierung von physikalichen Pulvermischungen oder übliche Granulationstechniken hergestellt werden und die ebenfalls die genannten Nachteile aufweisen.

Aufgabe dieser Erfindung war, ein Verfahren zu finden, mit dem diese Nachteile vermieden werden, das folglich zu sehr reproduzierbaren Freisetzungen und mechanisch sehr stabilen Darreichungsformen führt. Es sollte zudem einfach und sehr reproduzierbar durchzuführen sein.

Die Aufgabe wurde erfindungsgemäß gelöst, durch ein Verfahren gemäß Anspruch 1 zur Herstellung fester oraler Darreichungsformen mit kontrollierter Wirkstofffreisetzung, enthaltend eine Mischung aus
a) mindestens einen Wirkstoff,
b) eine vorformulierte Mischung aus Polyvinylacetat und Polyvinylpyrrolidon, welches dadurch gekennzeichnet ist, dass die Mischung aus den Komponenten a) und b) durch gemeinsame Verarbeitung von a) und b) in einem Extruder bei Temperaturen zwischen 80 und 180°C erfolgt, wobei die Extrusion bei einem Druck von 2 bis 25 MPa, bevorzugt 3 bis 20 MPa, besonders bevorzugt 5 bis 15 MPa erfolgt.

Die Porosität der extrudierten Mischung liegt bei kleiner als 10 Vol.-%, bevorzugt kleiner 5 Vol.-%, besonders bevorzugt kleiner 1 Vol.-%.

Weiterhin können als Komponenten c) gegebenenfalls wasserlösliche Polymere oder lipophile Zusatzstoffe eingesetzt werden. Als Komponenten d) können gegebenenfalls weitere übliche Hilfsstoffe eingesetzt werden. Diese Komponenten c) und/oder d) werden ebenfalls in Mischung mit den Komponenten a) und b) im Extruder eingearbeitet.

Kontrollierte Freisetzung im Sinne der vorliegenden Erfindung bedeutet insbesondere eine verlangsamte Freisetzung (auch als "retardierte" Freisetzung bezeichnet), die im englisch/amerikanischen Sprachgebrauch auch als "sustained release" bezeichnet wird. Erfindungsgemäß werden in drei Stunden weniger als 80% des Wirkstoffs freigesetzt.

Die Summe der Mengen an a), b) und gegebenenfalls c) und d) beträgt 100 Gew.-%.

Als Komponente a) kann grundsätzlich jeder Wirkstoff, der unter den Verfahrensbedingungen stabil verarbeitbar ist, eingesetzt werden. Insbesondere werden Wirkstoffe verarbeitet, für die eine retardierte Freisetzung erwünscht ist. In welchen Mengen der Wirkstoff eingesetzt wird, hängt von der pharmazeutisch relevanten Dosierung des Wirkstoffs ab.

Als Beispiele seien hier die folgenden genannt:

Benzodiazepine, Antihypertensiva, Vitamine, Cytostatika, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglo-buline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytica, Betarezeptorenblocker, Calciumantagonisten, ACE-Hemmer, Arteriosklerosemittel, Antiphlogistika, Antikoagulatia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel.

Besonders gut eignet sich das Verfahren für Wirkstoffe mit einer Wasserlöslichkeit bei 25 °C von kleiner 20 mg/ml, insbesondere kleiner 10mg/ml. Aber auch Wirkstoffe mit einer besseren Löslichkeit in Wasser, beispielsweise bis 100 mg/ml können mit Hilfe des erfindungsgemäßen Verfahrens verarbeitet werden.

Als Komponente b) wird eine vorformulierte Mischung aus Polyvinylacetat und Polyvinylpyrrolidon eingesetzt, in der Polyvinylacetat und Polyvinylpyrrolidon mit einem Gewichtsverhältnis 6:4 bis 9:1, vorzugsweise im Bereich von 8:2, vorliegen. Die Komponente b) wird in den Extrudaten zu mindestens 20 Gew.-%, bezogen auf die Gesamtmenge des Extrudats, eingesetzt. Sie kann je nach Dosierung des Wirkstoffs bis zu 99.9 Gew.-% betragen.

Als Polyvinylacetat wird erfindungsgemäß ein Homopolymer des Vinylacetats mit einem bezeichnet. Die Polyvinylacetate können Molekulargewichte von 20.000 bis 1.000.000 aufweisen, bevorzugt ein Molekulargewicht von 450.000 Dalton. Als Polyvinylpyrrolidon wird erfindungsgemäß ein Homopolymer das N-Vinylpyrrolidons, insbesondere ein Polyvinylpyrrolidon mit einem K-Wert von Fikentscher von 30, bezeichnet. Geeignete vorformulierte Mischungen aus Polyvinylacetat und Polyvinylpyrrolidon K30 sind kommerziell erhältlich als Kollidon® SR der Firma BASF.

Die vorformulierte Mischung aus Polyvinylacetat und Polyvinylpyrrolidon ist erhältlich durch Lösen von Polyvinylpyrrolidon in einer feinteiligen wässrigen Dispersion aus Polyvinylacetat mit einer Teilchengröße der Polyvinylacetat-Teilchen von 100 bis 300 nm und anschließende Sprühtrocknung der so erhaltenen Mischung. Es kann sich auch empfehlen bei der Sprühtrocknung ein Puderungsmittel wie beispielsweise Siliciumdioxid zu verwenden. Dadurch dass sich die beiden Polymere in Wasser und auch während der Sprühtrocknung nicht mischen, liegen sie in Form eines zweiphasigen Systems, dass keine physikalische Mischung darstellt und ohne Zerstörung der Struktur nicht separiert werden könnte, vor.

Die zur Herstellung der vorformulierten Mischung verwendeten Polyvinylacetat-Dispersionen können neben dem Polyvinylacetat auch Schutzkolloide zur Stabilisierung der wässrigen Dispersion enthalten, beispielsweise Polyvinylalkohol, Polyvinylpyrrolidon, Natriumlaurylsulfat oder Mischungen davon. Geeignete feinteilige wässrige Polyvinylacetat-Dispersionen zur Herstellung der vorformulierten Mischung sind kommerziell erhältlich, beispielsweise als Kollicoat® SR30D der Firma BASF, eine wässrige Dispersion mit einem Feststoffgehalt von 30 Gew.-%, die, bezogen auf das Gesamtgewicht der Dispersion, 27 Gew.-% Polyvinylacetat, 2.7 Gew.-% Polyvinylpyrrolidon K30 und 0.3 Gew.-% Natriumlaurylsulfat enthält..

Als Komponenten c) können wasserlösliche Polymere oder lipophile Zusatzstoffe zugegeben werden.

Durch den Zusatz von niedrigviskosen, nichtquellenden wasserlöslichen Polymeren, wie Polyvinylalkoholen, Polyethylenglykolen, Polyoxyethylen-Polyoxypropylen-Blockpolymerisaten, Polyvinylpyrrolidonen sowie Copolymeren von N-Vinylpyrrolidon, beispielsweise Vinylacetat-Vinylpyrrolidon-Copolymeren, oder Stärkederivaten, vorzugsweise Polyethylenglykolen, Polyvinylpyrrolidonen, Vinylacetat-Vinylpyrrolidon-Copolymeren oder Maltodextrinen oder Mischungen davon, kann die Wirkstofffreisetzung beschleunigt werden.

Durch Zugabe von wasserlöslichen, aber quellenden Polymeren als Komponenten c) kann die Freisetzung weiter variiert werden. Als wasserlösliche quellende Polymere können eingesetzt werden: Alginate, Pektine, Galactomannane, Carrageenane, Dextran, Curdlan, Pullulan, Gellan, Chitin, Gelatine, Xanthane, Hemicellulosen, Cellulosederivate wie Methylcellulose, Hydroxypropylmethylcellulose, Hydroxypropylcellulose, Hydroxyethylcellulose, Carboxymethylcellulose, Stärkederivate wie Carboxymethylstärke, Polyacrylsäure, Polymethacrylsäure, Acrylsäure-Methacrylsäure-Copolymere, Polyvinylalkohole, hochmolekulare Polyethylenglykole, Polyoxyethylen-Polyoxypropylen-Blockpolymerisate sowie hochmolekulare Polyvinylpyrrolidone oder Mischungen der genannten Stoffe.

Eine Verstärkung der Retardwirkung kann auch durch lipophile Zusatzstoffe als Komponenten c) erfolgen. Es ist wichtig, dass diese Stoffe in kleiner Korngröße eingesetzt werden, da sie in grober Form keine bzw. nur eine geringe Wirkung entfalten. Als lipophile Zusatzstoffe können sowohl Polymere als auch niedermolekulare Verbindungen verwendet werden. Bevorzugt sind allerdings die Polymere. Zu diesen Zusatzstoffen zählen: Cellulosederivate wie Ethylcellulose, Celluloseacetat, Celluloseacetatphthalat, Celluloseacetatsuccinat, Hydroxypropylmethylcelluloseacetatphthalat, Hydroxypropylmethylcelluloseacetatsuccinat, Acrylatester-Methacrylatester-Copolymerisate insbesondere Methylmethacrylat-Ethylacrylat-Copolymere, Ammonio-MethacrylateCopolymer Typ A und Typ B, Methacrylsäure-Acrylsäureester-Copolymere insbesondere Methacrylsäure-Ethylacrylat-Copolymere, Fettalkohole wie Stearylalkohol, Fettsäuren wie Stearinsäure, Fettsäureester und Fettalkoholester, Glyceride, Wachse, Lecithin.

Die fakultativen Komponenten c) können in Konzentrationen von 1 bis 40 %, bevorzugt von 2 bis 30 % bezogen auf das Tablettengesamtgewicht, eingesetzt werden. Dies ist vorteilhaft bei sehr niedrig dosierten Wirkstoffen, wo die zum Gerüstaufbau erforderliche Menge an formulierter Mischung aus Polyvinylacetat und Polyvinylpyrrolidon eine zu starke Retardierung mit sich bringen könnte. Ferner auch bei schwerlöslichen Wirkstoffen, bei denen niedrige Mengen an Retardierungsmittel zwar zu einer verzögerten Freisetzung führen, aber der Gerüstaufbau unvollständig ist, starken Schwankungen unterliegt und die mechanische Stabilität der Tabletten unzureichend ist. Dies ist insbesondere der Fall wenn der Wirkstoff schlecht verpressbar ist.

Als Komponenten d) können gegebenenfalls übliche pharmazeutische Hilfsstoffe mitverarbeitet werden. Dabei handelt es sich um Stoffe aus der Klasse der Füllstoffe, Weichmacher, Löslichkeitsvermittler, Bindemittel, Silikate sowie Spreng- und Adsorptionsmittel, Schmiermittel, Fließmittel, Farbstoffe, Stabilisatoren wie Antioxidantien, Netzmittel, Konservierungsmittel, Formentrennmittel, Aromen oder Süßstoffe, bevorzugt um Füllstoffe, Weichmacher und Löslichkeitsvermittler.

Als Füllstoffe können z.B. anorganische Füllstoffe wie Oxide von Magnesium, Aluminium, Silicium, Titan- oder Calciumcarbonat, Calcium- oder Magnesiumphosphate oder organische Füllstoffe wie Lactose, Saccharose, Sorbit, Mannit zugesetzt werden.

Als Weichmacher eignen sich beispielsweise Triacetin, Triethylcitrat, Glycerolmonostearat, niedermolekulare Polyethylenglykole oder Poloxamere.

Als Löslichkeitsvermittler eignen sich grenzflächenaktive Substanzen mit einem HLB-Wert (HydrophilicLipophilicBalance) größer 11, beispielsweise mit 40 Ethylenoxid-Einheitten ethoxiliertes hydriertes Ricinusöl (Cremophor® RH 40), mit 35 EthylenoxidEinheiten ethoxiliertes Ricinusöl (Cremophor eL), Polysorbat 80, Poloxamere oder Natriumlaurylsulfat.

Als Schmiermittel können Stearate von Aluminium, Calcium, Magnesium und Zinn, sowie Magnesiumsilikat, Silikone und ähnliche verwendet werden.

Als Fließmittel können beispielsweise Talk oder kolloidales Siliciumdioxid eingesetzt werden.

Als Bindemittel eignet sich zum Beispiel mikrokristalline Cellulose.

Als Sprengmittel können quervernetztes Polyvinylpyrrolidon oder quervernetzte Natriumcarboxymethylstärke sein. Stabilisatoren können sein Ascorbinsäure oder Tocopherol.

Farbstoffe sind z.B. Eisenoxide, Titandioxid, Triphenylmethanfarbstoffe, Azofarbstoffe, Chinolinfarbstoffe, Indigotinfarbstoffe, Carotinoide, um die Darreichungsformen einzufärben, Opakisierungsmittel wie Titandiodid oder Talkum, um die Lichtdurchlässigkeit zu erhöhen und um Farbstoffe einzusparen.

Die Komponente b) wird zusammen mit mindestens einem Wirkstoff und gewünschtenfalls den Komponenten c) und d) bei erhöhten Temperaturen im Extruder verarbeitet, Dabei werden Temperaturen der zu extrudierenden Masse von 80 bis 180 °C, besonders bevorzugt 100 bis 150 °C eingestellt.

Das erfindungsgemäße Verfahren erlaubt es, als Extruder Einschneckenmaschinen, kämmende Schneckenmaschinen oder auch Mehrwellenextruder, insbesondere Zweischnecken-Extruder, gleichsinnig oder gegensinnig drehend und gegebenenfalls mit Knetscheiben ausgerüstet, einzusetzen. Wenn gewünschtenfalls bei der Extrusion ein Lösungsmittel verdampft werden soll, sind die Extruder im Allgemeinen mit einem Verdampfungsteil ausgerüstet. Weiterhin sind die Extruder vorzugsweise mit einer Entgasungsvorrichtung ausgerüstet. Die Extruder können mit Zugabevorrichtung für pulverförmige oder flüssige Einsatzstoffe ausgerüstet sein. Besonders bevorzugt sind Zweischneckenextruder.

Der Austrag aus dem Extruder kann über Düsen oder Düsenplatten oder Lochplatten erfolgen, wobei der Austrag über Düsen bevorzugt ist. Die Düsen können auch beheizbar sein.

Die erfindungsgemäße Zubereitung verlässt den Extruder in Form eines Stranges oder Bandes und kann mittels eines nachgeschalteten Kalanders in einzeldosierte Formlinge zerteilt werden. Diese können eine runde, Oblong- oder Fussball-Form aufweisen. Weiterhin ist eine Formgebung zu festen Dosierungsformen mittels Spritzguss möglich. Es ist auch möglich den Strang mittels Heißabschlag, Unterwassergranulation oder Wasserringgranulation in Pellets oder Granulatkörner zu zerteilen, die auf pharmazeutisch üblichem Wege weiterverarbeitet werden können. Z. B. können sie mit oder ohne weitere übliche Hilfsstoffe in Kapseln gefüllt werden oder zu Tabletten verpresst werden. Auch eine vorherige Mahlung ist möglich.

Gemäß einer besonderen Ausführungsform der Erfindung wird anstelle eines Stranges ein Film mit einer Schichtdicke von 100 - 1000 µm extrudiert, aus dem anschließend kleinere Stücke ausgestanzt werden können. Diese können als sogenannte "Oral Strips" eingesetzt werden.

Durch die hohe Temperatur und den hohen Druck im Extruder wird der Wirkstoff innig mit der Polymergrundlage vermischt, sodass die Porosität sehr niedrig wird. Dadurch dass nahezu keine Lufteinschlüsse mehr vorhanden sind, die die Struktur der Formulierung verändern können, resultiert eine zum einen eine langsamere Freisetzung als bei herkömmlicher Verarbeitung mittels Schmelzgranulation oder Direkttablettierung und eine erheblich reproduzierbarere Freisetzung. Bei den aus dem oben erwähnten Stand der Technik bekannten Produkten der Schmelzgranulation handelt es sich um lockere Granulate, d.h. Strukturen aus mindestens 3 Phasen: Polymer, Arzneistoff und Luft. Die erfindungsgemäßen Extrudate bestehen im wesentlichen nur aus 2 Phasen, nämlich einer Polymerphase und Wirkstoff. In der Polymerphase liegt das Polyvinylacetat als kohärente Phase vor. Polyvinylpyrrolidon bildet Domänen in der kohärenten PVAc-Phase.

Zudem sind die Formlinge so hart aber zugleich auch zäh, dass sie mit bloßen Händen oder auch einfachen handwerklichen Mitteln wie einem Hammer nicht zu zerstören sind. Dadurch wird die missbräuchliche Verwendung der Tabletten, bei der die Tabletten zerkleinert werden und extrahiert werden, um den Arzneistoff zu gewinnen verhindert.

Bei diesem Verfahren kann durch die innige Vermischung unter Druck und hoher Temperatur der gesamte Arzneistoff oder auch nur ein gewisser Teil in der vorformulierten Mischung aus Polyvinylacetat und Polyvinylpyrrolidon gelöst werden, wodurch eine feste Lösung entsteht. Diese erklärt unter anderem die langsame Freisetzung und die bessere Reproduzierbarkeit. Wenn sich der gesamte Arzneistoff in der vorformulierten Mischung aus Polyvinylacetat und Polyvinylpyrrolidon löst, liegt nur eine Phase vor. Ferner besteht ein großer Vorteil des erfindungsgemäßen Verfahrens darin, dass Wirkstoffe sehr unterschiedlicher Teilchengröße eingesetzt werden können. Dies ist zum Beispiel bei der Direkttablettierung und der Schmelzgranulation nicht möglich, da feine Teilchen die Fließfähigkeit in der Direkttablettierung stark herabsetzen bzw. in der Schmelzgranulation die größeren klebrig gewordene Polymerteilchen abpudern, so dass kein Granulationseffekt auftritt.

Besonders geeignet ist das Verfahren für schwerlösliche Arzneistoffe, da diese in der vorformulierten Mischung aus Polyvinylacetat und Polyvinylpyrrolidon gelöst werden können, so dass die Freisetzung nicht mehr vom partikulären Zustand des Arzneistoffes abhängt. Bei partikulär vorliegenden schwerlöslichen Arzneistoffen üben die Teilchengröße, die Oberfläche, die Benetzbarkeit des Teilchens und die Modifikation einen sehr großen Einfluss auf die Freisetzung aus.

### Beispiele

### Verwendete Abkürzungen:

PVAc: Polyvinylacetat
PVP: Polyvinylpyrrolidon K30
VA 64: Kollidon® VA 64 (BASF), Copolymer aus N-Vinylpyrrolidon und Vinylacetat im Gewichtsverhältnis 6:4

Soweit nicht anders angegeben stellen %-Angaben Gewichtsprozente dar.

Zur Untersuchung der Eignung der Extrusion für die Herstellung von Matrixretardtabletten wurden Rezepturen mit Theophyllin und der vorformulierten Mischung aus (80 Gew.-% PVAc, 19 Gew.-% PVP, 0.8 Gew.-% Natriumlaurylsulfat und 0.2 Gew.-% Siliciumdioxid (Kollidon® SR) sowie mit und ohne Zusatz von VA 64 zur Steuerung des Freisetzungsprofils eingesetzt. Die genaue Zusammensetzung ist in der nachstehenden Tabelle 1 angegeben.

| Tabelle 1 | | | | |
|---|---|---|---|---|
| | | | | |

| Beispiel Nr. | 1 | 2 | 3 | 4 |
|---|---|---|---|---|
| Theophyllin | 60% | 50% | 50% | 60% |
| Kollidon SR | 40% | 40% | 30% | 24% |
| VA64 | 0% | 10% | 20% | 16% |

Für die Extrusion wurde ein Zweischneckenextruder ZSK 25 der Firma Werner & Pfleiderer eingesetzt. Der Durchmesser der Schnecken betrug 25 mm und das Verhältnis Schneckenlänge zu Durchmesser 34. Der Austrag der Polymer- Wirkstoffschmelze erfolgte über eine Düsenleiste mit 3 Bohrungen mit an 1,5mm bzw. 2 mm Durchmesser. Insgesamt wurden 8 beheizbare Elemente verwendet.

Die Extrusionsbedingungen und eingesetzten Temperaturprofile sind in Tabelle 32 aufgeführt.

| Tabelle 2 | | | | | |
|---|---|---|---|---|---|
| Charge | | 1 | 2 | 3 | 4 |
| Massedruck | [MPa] | 9,3 | 6,7 | 7,0 | 5,9 |
| Leistung | [kW] | 2.0 | 1.7 | 1.6 | 1.6 |
| Drehzahl | [U/min] | 197 | 197 | 199 | 197 |
| Düsendurchmesser | [mm] | 2 | 2 | 1.5 | 2 |
| Anzahl Düsen | | 3 | 2 | 3 | 2 |
| Drehmoment | [A] | 10.3 | 8.4 | 8.1 | 8.0 |
| Heizzone 1 | [°C] | 140 | 140 | 140 | 141 |
| Heizzone 2 | [°C] | 139 | 139 | 138 | 140 |
| Heizzone 3 | [°C] | 141 | 139 | 141 | 140 |
| Heizzone 4 | [°C] | 142 | 139 | 140 | 138 |
| Heizzone 5 | [°C] | 143 | 141 | 140 | 139 |
| Heizzone 6 | [°C] | 137 | 144 | 141 | 143 |
| Heizzone 7 | [°C] | 154 | 148 | 147 | 163 |
| Heizzone 8 | [°C] | 151 | 148 | 150 | 157 |

Die erhaltenen Extrudate wurden anschließend gemahlen und mit jeweils 0,5% Magnesiumstearat versetzt. Für die Tablettierung von 400 mg schweren Tabletten wurden 12 mm facettierte Stempel und eine Presskraft von 18kN verwendet.

Tabletten, die mit gemahlenem Extrudatpulver hergestellt wurden, weisen tendenziell eine geringere Bruchfestigkeit und Zugfestigkeit (Tensile Strength) auf. Bezüglich der Friabilität entsprechen die Tabletten denen aus der Direkttablettierung (siehe Tabelle 3).

**Tabelle 3**

| charge | 1 | | 2 | | 3 | | 4 | |
|---|---|---|---|---|---|---|---|---|
| Prozess | Pulver | Extrudat | Pulver | Extrudat | Pulver | Extrudat | Pulver | Extrudat |
| Bruchfestigkeit [N] | 192 ± 24 | 196 ± 5 | 223 ± 8 | 175 ± 6 | 139 ± 5 | 107 ± 5 | 175 ± 6 | 139 ± 5 |
| Tensile strength [N/mm²] | 3.5 ± 0.4 | 3.5 ± 0.1 | 3.9 ± 0.1 | 3.2 ± 0.1 | 2.5 ± 0.1 | 1.9 ± 0.1 | 3.2 ± 0.1 | 2.5 ± 0.1 |
| Friabilität [%] | 0.09 | 0.06 | 0.09 | 0.07 | 0.04 | 0.08 | 0.07 | 0.04 |

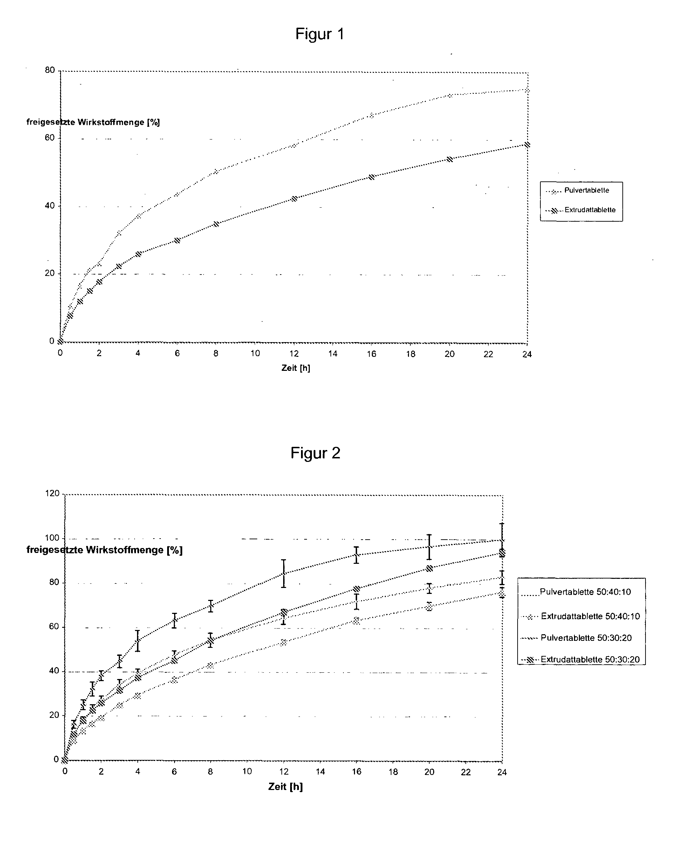

Anhand der Freisetzungsprofile der Chargen lässt sich deutlich der Einfluss der Extrusion auf die Wirkstofffreigabe aus den Matrices erkennen. Bei Einsatz von 60% Wirkstoff und Kollidon SR wird durch Extrusion eine deutlich langsamere Freisetzung erzielt (Tabelle 4 und Figur 1)

| Tabelle 4 | | |
|---|---|---|
| Beispiel Nr. | 1 | |
| Theophyllin | 60% | |
| Kollidon SR | 40% | |
| VA64 | 0% | |

| Zeit | Pulver Freisetzung [%] | Extrudat Freisetzung [%] |
|---|---|---|
| 0.5 | 10.6 | 7.81 |
| 1 | 16.6 | 12.01 |
| 1.5 | 21.3 | 15.17 |
| 2 | 23.0 | 17.76 |
| 3 | 32.2 | 22.34 |
| 4 | 37.2 | 25.92 |
| 6 | 43.7 | 30.08 |
| 8 | 50.5 | 34.96 |
| 12 | 58.4 | 42.47 |
| 16 | 67.3 | 49.00 |
| 20 | 73.2 | 54.30 |
| 24 | 75.0 | 58.81 |

Durch Zugabe von VA 64 zur Rezeptur wird die Freisetzung beschleunigt (siehe Tabelle 5 und Figur 2). Die Extrudattabletten weisen eine deutliche langsamere und gleichmäßigere Freisetzung auf.

| Tabelle 5: Wirkstoff-Freisetzung | | | | |
|---|---|---|---|---|
| Beispiel Nr. | 2 | | 3 | |
| Thcophyllin | 50% | | 50% | |
| Kollidon SR | 40% | | 30% | |
| VA64 | 10% | | 20% | |

| Zeit | Pulver [%] | Extrudat [%] | Pulver [%] | Extrudat [%] |
|---|---|---|---|---|
| 0.5 | 11.5 ± 0.3 | 9.1 ± 0.3 | 16.3 ± 1.8 | 11.8 ± 0.2 |
| 1 | 18.3 ± 1.2 | 13.3 ± 0.2 | 25.1 ± 2.2 | 17.9 ± 0.2 |
| 1.5 | 23.6 ± 1.5 | 16.8 ± 0.2 | 32.3 ± 3.1 | 22.5 ± 0.6 |
| 2 | 27.5 ± 1.6 | 19.4 ± 0.3 | 38.3 ± 2.2 | 25.9 ± 0.7 |
| 3 | 34.2 ± 2.3 | 25.2 ± 0.2 | 44.7 ± 2.8 | 31.6 ± 0.6 |
| 4 | 39.3 ± 1.9 | 29.5 ± 0.6 | 54.0 ± 4.8 | 37.2 ± 0.6 |
| 6 | 47.8 ± 1.8 | 36.5 ± 0.8 | 63.2 ± 3.3 | 45.1 ± 0.4 |
| 8 | 54.3 ± 3.3 | 43.3 ± 0.4 | 69.8 ± 2.6 | 54.2 ± 0.4 |
| 12 | 64.3 ± 2.6 | 53.6 ± 1.0 | 84.5 ± 6.1 | 67.1 ± 1.4 |
| 16 | 71.9 ± 3.5 | 63.4 ± 1.1 | 92.8 ± 3.7 | 77.8 ± 0.2 |
| 20 | 78.1 ± 2.2 | 69.9 ± 1.8 | 96.7 ± 5.6 | 87.1 ± 0.7 |
| 24 | 83.1 ± 3.0 | 76.3 ± 2.2 | 99.9 ± 7.5 | 94.1 ± 1.7 |

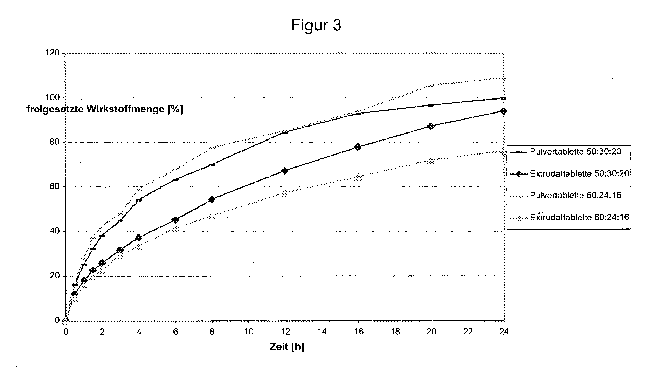

Wird das Verhältnis Kollidon SR : VA 64 konstant gehalten, zeigen die Extrudattabletten eine deutliche langsamere und gleichmäßigere Freisetzung mit einer geringeren Schwankungsbreite (Tabelle 6, Figur 3). Ein weiterer Vorteil der Extrudate liegt darin, dass ab 50 % Freisetzung die Kurve nicht abflacht, sondern linear einer Kinetik nullter Ordnung folgt. Es ist weiterhin möglich, die Freisetzung durch Erhöhung des Kollidon VA 64 Anteils zu beschleunigen, wohingegen bei mit Pulvermischungen direkttablettierten Tabletten nahezu identische, schnelle Freisetzungsprofile erhalten werden, die zudem eine hohe Schwankungsbreite aufweisen.

| Tabelle 6 | | | | |
|---|---|---|---|---|
| Theophyllin | 50% | | 60% | |
| Kollidon SR | 30% | | 24% | |
| Kollidon VA64 | 20% | | 16% | |

| Zeit [h] | Pulver [%] | Extrudat [%] | Pulver [%] | Extrudat [%] |
|---|---|---|---|---|
| 0.5 | 16.3 | 11.8 | 17.0 | 10.0 |
| 1 | 25.1 | 17.9 | 28.1 | 15.5 |
| 1.5 | 32.3 | 22.5 | 37.2 | 19.9 |
| 2 | 38.3 | 25.9 | 42.5 | 22.7 |
| 3 | 44.7 | 31.6 | 47.7 | 29.5 |
| 4 | 54.0 | 37.2 | 58.8 | 33.4 |
| 6 | 63.2 | 45.1 | 67.6 | 41.5 |
| 8 | 69.8 | 54.2 | 77.3 | 47.0 |
| 12 | 84.5 | 67.1 | 85.1 | 57.2 |
| 16 | 92.8 | 77.8 | 93.8 | 64.2 |
| 20 | 96.7 | 87.1 | 105.3 | 71.9 |
| 24 | 99.9 | 94.1 | 108.9 | 76.2 |

## Patentansprüche

1. Verfahren zur Herstellung fester oraler Darreichungsformen mit kontrollierter Wirkstofffreisetzung, enthaltend eine Mischung aus
a) mindestens einen Wirkstoff; und
b) eine vorformulierte Mischung aus Polyvinylacetat und Polyvinylpyrrolidon, wobei die vorformulierte Mischung erhältlich ist durch Lösen von Polyvinylpyrrolidon in einer feinteiligen wässrige Dispersion von Polyvinylacetat mit einer Teilchengröße der Polyvinylacetat-Teilchen von 100 bis 300 nm und anschliessende Sprühtrocknung,
**dadurch gekennzeichnet, dass** die Mischung durch gemeinsame Verarbeitung der Komponenten a) und b) in einem Extruder bei Temperaturen zwischen 80° und 180°C erhalten wird, wobei die Extrusion bei einem Druck von 2 bis 25 MPa erfolgt.

2. Verfahren nach Anspruch 1, **dadurch gekennzeichnet, dass** als zusätzliche Komponenten c) wasserlösliche Polymere oder lipophile Zusatzstoffe in die Mischung aus den Komponenten a) und b) eingearbeitet werden.

3. Verfahren nach Anspruch 1 oder 2, **dadurch gekennzeichnet, dass** als Komponenten d) weitere übliche Hilfsstoffe in die Mischung eingearbeitet werden.

4. Verfahren nach einem der Ansprüche 1 bis3, **dadurch gekennzeichnet, dass** die Extrusion bei einem Druck von 3 bis 20 MPa erfolgt.

5. Verfahren nach einem der Ansprüche 1 bis 4, **dadurch gekennzeichnet, dass** die Extrusion bei einem Druck von 5 bis 15 MPa erfolgt.

6. Verfahren nach einem der Ansprüche 1 bis 5, **dadurch gekennzeichnet**, die Porosität der extrudierten Mischung kleiner als 10 Vol.- % beträgt.

7. Verfahren nach einem der Ansprüche 1 bis 6, **dadurch gekennzeichnet dass** die Porosität kleiner 5 Vol.-% beträgt

8. Verfahren nach einem der Ansprüche 1 bis 7, **dadurch gekennzeichnet dass** die Porosität kleiner 1 Vol.-% beträgt.

9. Verfahren nach einem der Ansprüche 1 bis 8, **dadurch gekennzeichnet, dass** die Mischung in Form eines Strangs extrudiert wird.

10. Verfahren nach einem der Ansprüche 1 bis 9, **dadurch gekennzeichnet dass** der extrudierte Strang zu einzeldosierten, tablettenähnlichen Formlingen kalandriert wird.

11. Verfahren nach einem der Ansprüche 1 bis 10, **dadurch gekennzeichnet dass** der extrudierte Strang zu Pellets oder Granulatteilchen zerkleinert wird.

12. Verfahren nach einem der Ansprüche 1 bis 11, **dadurch gekennzeichnet, dass** der zerkleinerte, extrudierte Strang in Kapseln oder Sachets gefüllt oder zu Tabletten verpresst wird.

13. Verfahren nach einem der Ansprüche 1 bis 8, **dadurch gekennzeichnet dass** ein Film mit einer Schichtdicke von 100 - 1000 µm extrudiert wird, aus dem direkt anschließend kleine Filmstücke für die orale Applikation ausgestanzt werden.

14. Verfahren nach einem der Ansprüche 1 bis 13, **dadurch gekennzeichnet dass** Wirkstoffe mit einer Wasserlöslichkeit von kleiner 20 mg/ml eingesetzt werden.

15. Verfahren nach einem der Ansprüche 1 bis 14 , **dadurch gekennzeichnet dass** Wirkstoffe mit einer Wasserlöslichkeit kleiner 10 mg/ml eingesetzt werden

16. Feste orale Darreichungsformen mit kontrollierter Freisetzung, enthaltend eine extrudierte Mischung gemäß einem der Ansprüche 1 bis 15.

## Claims

1. A process for producing solid oral dosage forms with controlled active ingredient release, comprising a mixture of
a) at least one active ingredient, and
b) a preformulated mixture of polyvinyl acetate and polyvinylpyrrolidone, the preformulated mixture being obtainable by dissolving polyvinylpyrrolidone in a fine-particle aqueous dispersion of polyvinyl acetate with a particle size of the polyvinyl acetate particles of from 100 to 300 nm, and subsequently spray drying,
wherein the mixture is obtained by joint processing of components a) and b) in an extruder at temperatures between 80° and 180°C, the extrusion taking place under a pressure of from 2 to 25 MPa.

2. The process according to claim 1, wherein water-soluble polymers or lipophilic additives are incorporated as additional components c) into the mixture of components a) and b).

3. The process according to claim 1 or 2, wherein further conventional excipients are incorporated as components d) into the mixture.

4. The process according to any of claims 1 to 3, wherein the extrusion takes place under a pressure of from 3 to 20 MPa.

5. The process according to any of claims 1 to 4, wherein the extrusion takes place under a pressure of from 5 to 15 MPa.

6. The process according to any of claims 1 to 5, wherein the porosity of the extruded mixture is less than 10% by volume.

7. The process according to any of claims 1 to 6, wherein the porosity is less than 5% by volume.

8. The process according to any of claims 1 to 7, wherein the porosity is less than 1% by volume.

9. The process according to any of claims 1 to 8, wherein the mixture is extruded in the form of a strand.

10. The process according to any of claims 1 to 9, wherein the extruded strand is calendered to give single-dose, tablet-like shaped articles.

11. The process according to any of claims 1 to 10, wherein the extruded strand is reduced in size to pellets or granule particles.

12. The process according to any of claims 1 to 11, wherein the extruded strand which has been reduced in size is packed into capsules or sachets or compressed to tablets.

13. The process according to any of claims 1 to 8, wherein a film with a layer thickness of 100-1000 µm is extruded, from which small pieces of film for oral administration are cut out directly thereafter.

14. The process according to any of claims 1 to 13, wherein active ingredients with a solubility in water of less than 20 mg/ml are employed.

15. The process according to any of claims 1 to 14, wherein active ingredients with a solubility in water of less than 10 mg/ml are employed.

16. A solid oral dosage form with controlled release comprising an extruded mixture according to any of claims 1 to 15.

## Revendications

1. Procédé pour la préparation de formes d'administration orales solides dotées d'une libération contrôlée des substances actives, contenant un mélange
a) d'au moins une substance active ; et
b) d'un mélange préformulé de poly(acétate de vinyle) et de polyvinylpyrrolidone, le mélange préformulé pouvant être obtenu par dissolution de polyvinylpyrrolidone dans une dispersion aqueuse finement divisée de poly(acétate de vinyle) présentant une grosseur des particules de poly(acétate de vinyle) de 100 à 300 nm et par séchage par pulvérisation consécutif,
**caractérisé en ce que** le mélange est obtenu par traitement commun des composants a) et b) dans une extrudeuse à des températures entre 80°C et 180°C, l'extrusion ayant lieu à une pression de 2 à 25 MPa.

2. Procédé selon la revendication 1, **caractérisé en ce qu'**on incorpore, comme composants supplémentaires c), des polymères solubles dans l'eau ou des additifs lipophiles dans le mélange des composants a) et b).

3. Procédé selon la revendication 1 ou 2, **caractérisé en ce qu'**on incorpore comme composants d) d'autres adjuvants usuels dans le mélange.

4. Procédé selon l'une quelconque des revendications 1 à 3, **caractérisé en ce que** l'extrusion a lieu à une pression de 3 à 20 MPa.

5. Procédé selon l'une quelconque des revendications 1 à 4, **caractérisé en ce que** l'extrusion a lieu à une pression de 5 à 15 MPa.

6. Procédé selon l'une quelconque des revendications 1 à 5, **caractérisé en ce que** la porosité du mélange extrudé est inférieure à 10% en volume.

7. Procédé selon l'une quelconque des revendications 1 à 6, **caractérisé en ce que** la porosité est inférieure à 5% en volume.

8. Procédé selon l'une quelconque des revendications 1 à 7, **caractérisé en ce que** la porosité est inférieure à 1% en volume.

9. Procédé selon l'une quelconque des revendications 1 à 8, **caractérisé en ce que** le mélange est extrudé sous forme d'un brin.

10. Procédé selon l'une quelconque des revendications 1 à 9, **caractérisé en ce que** le brin extrudé est calandré en pièces façonnées à dose unique, analogues à des comprimés.

11. Procédé selon l'une quelconque des revendications 1 à 10, **caractérisé en ce que** le brin extrudé est broyé en pellets ou en particules de granulat.

12. Procédé selon l'une quelconque des revendications 1 à 11, **caractérisé en ce que** le brin extrudé, broyé est introduit dans des capsules ou des sachets ou pressé en comprimés.

13. Procédé selon l'une quelconque des revendications 1 à 8, **caractérisé en ce qu'**on extrude un film présentant une épaisseur de couche de 100-1000 µm, dans lequel on découpe ensuite directement de petits morceaux de film pour une administration par voie orale.

14. Procédé selon l'une quelconque des revendications 1 à 13, **caractérisé en ce qu'**on utilise des substances actives présentant une solubilité dans l'eau inférieure à 20 mg/ml.

15. Procédé selon l'une quelconque des revendications 1 à 14, **caractérisé en ce qu'**on utilise des substances actives présentant une solubilité dans l'eau inférieure à 10 mg/ml.

16. Formes d'administration orales solides dotées d'une libération contrôlée, contenant un mélange extrudé selon l'une quelconque des revendications 1 à 15.
